# NOUVEAU FASCICULE DE BREVET EUROPEEN

(11) **EP 3 818 973 B2**
(45) Date de publication et mention de la décision concernant l'opposition: **02.12.2026**
(45) Mention de la délivrance du brevet: 07.02.2024
(21) Numéro de dépôt: 20205977.0
(22) Date de dépôt: 05.11.2020
(51) Int. Cl.: A61K 8/49, A61K 8/73, A61Q 17/04

(54) **COMPOSITION SOLAIRE RESISTANTE A L'EAU A HAUTE TENEUR DE TRIAZINES**
WASSERFESTE SONNENSCHUTZZUSAMMENSETZUNG MIT EINEM HOHEN GEHALT AN TRIAZINEN
WATER-RESISTANT SOLAR COMPOSITION WITH HIGH TRIAZINE CONTENT

(30) Priorité: 05.11.2019 FR 1912404
(43) Date de publication de la demande: 12.05.2021
(73) Titulaire: Thorel, Jean-Noël, 75014 Paris (FR); NAOS Institute of Life Science, 13290 Aix-en-Provence (FR)
(72) Inventeur: THOREL, Jean-Noël, 75014 PARIS (FR)
(74) Mandataire: Nederlandsch Octrooibureau

(56) Documents cités:
- GB-A- 2 439 618
- DATABASE GNPD [online] MINTEL; 9 August 2017 (2017-08-09), ANONYMOUS: "Daily Defense DD Cream SPF 30", XP055708359, retrieved from www.gnpd.com Database accession no. 5006587

## Description

### Domaine de l'invention

La présente invention concerne une composition, avantageusement cosmétique ou dermatologique, à haute teneur en filtres solaires organiques dérivés de triazine et son utilisation pour protéger la peau du rayonnement ultraviolet.

### Etat antérieur de la technique

Le rayonnement ultraviolet (UV) se compose de lumière de longueur d'onde comprise entre 100 nm et 400 nm, traditionnellement répartie en 3 sous-groupes : les UV-A (400-315 nm), les UV-B (315-280 nm) et les UV-C (280-100 nm). Les UV-C, de courte longueur d'onde, sont les UV les plus énergétiques et les plus nocifs. Ils sont filtrés par la couche d'ozone de l'atmosphère et n'atteignent pas la surface de la Terre en quantité considérable. La plupart des dégâts sur la peau restent causés par les UV-A et les UV-B.

Les UV-B induisent la production du pigment naturel de la peau appelé mélanine. Ce qui est à l'origine du phénomène communément appelé le bronzage. Les UV-B stimulent également les cellules pour qu'elles produisent un épiderme plus épais. Les réactions décrites ci-avant constituent un mécanisme de défense de l'organisme contre le rayonnement UV.

L'énergie importante des UV-B génère des désordres moléculaires (altération de l'ADN et dommages aux protéines, notamment leur carbonylation) qui, à long terme, saturent et enrayent le système de réparation de 1'ADN nucléaire. Cela entraîne des mutations permanentes dans le génome des cellules atteintes, qui sont à l'origine de cancers cutanés. Il s'agit alors d'une toxicité directe des UV-B. Quant aux UV-A, il est connu qu'ils pénètrent dans les couches profondes de la peau, où ils produisent des effets délétères notamment au sein du tissu conjonctif et sur les vaisseaux sanguins. Ils sont notamment à l'origine du phénomène nommé héliodermie, c'est-à-dire le vieillissement actinique prématuré de la peau.

De surcroit, bien que les UV-B soient les principaux responsables des cancers cutanés, les UV-A ont également une contribution indirecte à ce type d'atteinte. En effet, les UV-A et les UV-B sont à l'origine de la production de radicaux libres, notamment les ROS (*Reactive Oxygen Species* ou espèces réactives de l'oxygène (ERO)). Il s'agit de molécules très instables ayant une demi-vie très courte, de l'ordre de la nanoseconde à la milliseconde. Les ROS sont capables d'endommager les structures intracellulaires (ADN, membranes, protéines intracellulaires...) ainsi que les structures extracellulaires (les composants de la matrice extracellulaire comme les fibres de collagène...). Les ROS exercent également une action nocive indirecte, en provoquant l'oxydation des lipides membranaires, ce qui induit la formation des espèces réactives carbonylés qui contribuent à amplifier les dégâts tissulaires et cellulaires. Les mêmes dommages sont également causés et même amplifiés par la pollution environnementale, qui génère également des radicaux libres responsables du vieillissement cutané.

Il est donc nécessaire de lutter contre les effets nocifs du rayonnement UV, notamment UV-A et UV-B, en tenant compte des différences interindividuelles telles que le type de peau (phototype) et les temps d'exposition au soleil.

Une stratégie de lutte contre les dommages causés par le soleil sur la peau est la protection externe. Elle s'effectue en filtrant le rayonnement UV ou en occultant les zones exposées au soleil. Le recouvrement des zones exposées assure une photoprotection totale, mais n'est pas toujours possible ou pratique. Pour cette raison, des compositions solaires ont été développées afin d'assurer une photoprotection des zones sujettes au rayonnement UV. Ces compositions se présentent sous la forme de lotion, huile, émulsion de type huile-dans-eau ou eau-dans-huile, mousse, gel, stick ou spray. Elles contiennent un support cosmétiquement acceptable et un ou plusieurs filtres chimiques ou écrans minéraux à des concentrations diverses.

Les associations de filtres ou écrans minéraux, qui ont des spectres d'absorption différents, et les quantités mises en oeuvre, sont sélectionnées en fonction du niveau de protection solaire recherché et/ou de la zone du corps à protéger.

Les écrans solaires minéraux, parmi lesquels on peut citer les oxydes de zinc et les dioxydes de titane, protègent des UV en reflétant la lumière solaire. Ils agissent donc comme des miroirs localisés à la surface de la peau. Les écrans minéraux, du moins sous la formule micrométrique, sont surs pour l'utilisation humaine et ne comportent aucun risque pour la santé.

Cependant, ils présentent d'autres inconvénients :
- les écrans minéraux ont tendance à dessécher la peau et à s'étaler avec difficulté, en laissant des trainées blanches sur la peau qui peuvent se révéler très inesthétiques ;
- l'appréciation galénique des compositions à base d'écrans minéraux est souvent médiocre, notamment dans les compositions exemptes de silicones.

Ces caractéristiques techniques des écrans minéraux peuvent limiter l'observance de produits à base de ce système de filtration de la lumière UV par les utilisateurs.

Les filtres chimiques sont des composés organiques qui absorbent la lumière en créant sur la peau une couche filtrante qui neutralise les rayons UV. Ainsi, lorsque la molécule de filtre solaire reçoit une radiation UV, elle passe dans un état excité en absorbant l'énergie du rayonnement puis retourne dans son état stable. Par ce mécanisme, la radiation lumineuse est convertie en chaleur et dissipée. Les filtres chimiques agissent donc de la même façon que la mélanine, le pigment photoprotecteur naturel de la peau.

Les filtres solaires chimiques présentent plusieurs aspects avantageux. Ils agissent de façon synergique en couvrant des plages de longueur d'onde différentes, ce qui permet d'obtenir une protection uniforme dans les UV-B et UV-A voire même jusqu'au visible à haute énergie. Les filtres chimiques peuvent également se stabiliser, et notamment se photostabiliser réciproquement, ce qui peut contribuer à assurer la rémanence de la photoprotection.

Ces caractéristiques des filtres organiques ont été à la base de leur succès : la plupart des produits solaires, notamment les produits solaires à haute protection caractérisés par un facteur de protection solaire (FPS ou SPF pour la dénomination anglaise « Sun Protection Factor »), supérieur à 30 sont formulés en employant des associations de filtres organiques, éventuellement complémentées par des quantités mineures d'écrans minéraux.

Parmi les filtres organiques autorisés en Europe qui sont considérés, à ce jour, parfaitement surs pour l'utilisation chez l'homme, on retrouve des filtres solaires dérivés de triazine, notamment de 1,3,5-triazine, 1,2,4-triazine et 1,2,4-triazine. Ce type de filtres solaires dérivés de triazine correspond, par exemple, aux désignations INCI suivantes : ethylhexyl triazone, tris-biphenyl triazine, diethylhexyl butamido triazone, phenylene bis-diphenyltriazine, ethylhexyl bis-isopentylbenzoxazolylphenyl melamine ou encore bis-ethylhexyloxyphenol methoxyphenyl triazine.

Ce groupe de filtres solaires organiques possède des propriétés photoprotectrices uniques car les dérivés de triazine couvrent de larges portions du spectre UV-A et UV-B. En outre, ils sont extrêmement stables notamment parce qu'ils peuvent se photostabiliser réciproquement et participent également à la stabilisation d'autres filtres solaires. Les filtres solaires dérivés de triazine sont bien tolérés au niveau cutané et adaptés à la formulation de produits à appliquer sur peau saine mais également sur peaux lésées, fragilisées ou pathologiques car ces composés sont dépourvus de propriétés allergisantes ou irritantes.

Cependant, il est techniquement difficile de produire des compositions solaires pourvues à la fois de bonnes propriétés galéniques, d'une capacité de résistance à l'eau et de tolérance cutanée élevée et comprenant également des hautes teneurs de filtres solaires dérivés de triazine.

Au sens de l'invention, une haute teneur en filtres solaires dérivés de triazine désigne une quantité strictement supérieure à 10% en poids total de la composition, avantageusement supérieure à 12%.

Les filtres solaires dérivés de triazines se solubilisent dans les corps gras, notamment dans les esters. Une forme galénique adaptée aux compositions comprenant de hautes teneurs de triazine sont les huiles anhydres. Ce type de formulation est efficace en termes de photoprotection et caractérisé par une bonne résistance à l'eau. Cependant, les huiles sont perçues comme sensoriellement désagréables, grasses et collantes par la plupart des utilisateurs.

Pour encourager les utilisateurs à s'appliquer suffisamment de produits filtrants les UV et protéger la peau de manière adéquate, il est donc préférable de proposer des compositions comprenant une phase grasse et une phase aqueuse, par exemple les émulsions E/H, H/E, les émulsions triples ou encore les gels crème. La présence de deux phases dispersées l'une dans l'autre permet d'obtenir des compositions pourvues des bonnes propriétés galéniques et d'un toucher agréable. Ces compositions peuvent également contribuer à l'hydratation de la peau grâce à leur contenu en eau. En outre, la phase aqueuse permet d'incorporer à la formulation des principes actifs ainsi que des filtres solaires hydrophiles, en améliorant l'efficacité globale et les propriétés dermatologiques des produits solaires.

Cependant, une limite à l'utilisation de ces formulations, notamment les gels aqueux à base d'hydrocolloïdes, est qu'elles ne permettent pas de disperser la phase grasse. Une solution est la formulation sous forme de gel crème, c'est-à-dire une composition biphasée ou biphasique comprenant des gélifiants. Toutefois, les compositions de type gel crème se prêtent mal à l'incorporation de hautes teneurs de filtres solaires dérivés de triazine car les gélifiants peuvent disperser seulement de petites quantités de phase grasse.

Les émulsions, quant à elles, permettent l'incorporation de quantités plus importantes de phase grasse, mais souffrent d'autres inconvénients. Par exemple, les tensioactifs nécessaires pour la fabrication des émulsions peuvent irriter la peau. De ce fait, leur utilisation n'est pas toujours indiquée pour la formulation de produits solaires destinés à la protection des peaux notamment lésées ou pathologiques. En effet, dans ce contexte, les produits solaires doivent être caractérisés par une innocuité totale.

Le document GB 2 439 618 concerne composition de soins de la peau sous forme d'une émulsion comprenant : a) un composant d'écran solaire comprenant au moins un écran solaire organique choisi dans le groupe constitué par la bis-éthylhexyloxyphénol, la méthoxyphényl triazine et le méthylène bis-benzotriazolyl tétraméthylbutylphénol ; b) un système hydratant comprenant de l'amidon ou un dérivé de celui-ci et un sel de composé quaternaire polymère ayant des propriétés humectantes ; et c) un système émulsifiant comprenant au moins un émulsifiant comprenant un émulsifiant anionique ou non ionique dans laquelle l'amidon ou un dérivé de l'amidon est également présent comme hydratant dans les compositions.

De surcroit, la présence de tensioactifs modifie le toucher de la composition et limite inévitablement leur résistance à l'eau, car en contact avec l'eau, les tensioactifs contribuent au lavage de la phase grasse. Ce problème, qui est particulièrement prononcé dans le cas de tensioactifs hydrophiles, oblige les utilisateurs à se réappliquer le produit solaire après chaque baignade ou activité sportive.

Il subsiste donc un besoin évident de mettre au point des compositions comprenant de hautes teneurs en filtres solaires dérivés de triazine possédant à la fois de bonnes propriétés galéniques, une tolérance cutanée et une résistance à l'eau accrues.

### Exposé de l'invention

Le Demandeur a constaté qu'une composition comprenant deux types d'amidon modifié en présence d'une phase aqueuse, d'une phase grasse, et de filtres solaires dérivés de triazine permet de répondre aux besoins mentionnés précédemment (solubilisation de hautes teneurs en filtres solaires dérivés de triazine, galénique, tolérance cutanée et résistance à l'eau). Notamment, la composition selon l'invention a pour avantage de présenter une résistance à l'eau accrue, avec un excellent maintien de la protection solaire (maintien du SPF) suite à l'exposition à l'eau.

Selon un premier aspect, l'invention concerne une composition solaire telle que définie dans la revendication 1 et cette composition pour utilisation pour la protection de la peau, les lèvres, les cheveux et/ou les muqueuses contre le rayonnement solaire ultraviolet.

L'hydroxypropyl starch phosphate, l'hydroxypropyl distarch phosphate et le sodium starch octenylsuccinate sont des amidons modifiés, c'est-à-dire des amidons qui ont subi des altérations chimiques ou physiques par rapport à leur état d'origine.

Les amidons selon l'invention peuvent être d'origine animale, végétale, ou biotechnologique.

Selon un mode de réalisation particulier, les amidons sont d'origine végétale, avantageusement obtenus par extraction à partir de maïs, de préférence des graines de maïs.

L'hydroxypropyl starch phosphate (numéro CAS 53124-00-8) est un type d'amidon modifié par greffage covalent des groupements hydroxypropyle. Les amidons hydroxypropylés sont connus à l'homme du métier et se retrouvent par exemple sous la désignation E1400 selon le système international de numérotation des additifs alimentaires (SIN).

Dans un autre mode de réalisation, la matière première répond à la désignation INCI hydroxypropyl distarch phosphate.

A titre d'exemple, les matières premières Structure^{®} 2143, Structure^{®} 6892, BTC^{®} et HVS^{®} commercialisées par la société NOURYON CHEMICALS, ou encore la matière première C*HiForm 12748 commercialisée par la société CARGILL INC, peuvent être utilisée comme source d'hydroxypropyl starch phosphate ou d'hydroxypropyl distarch phosphate.

Le sodium starch octenylsuccinate (numéro CAS 66829-29-6) est un amidon modifié obtenu par réaction de petites quantités d'anhydride octénylsuccinique avec un amidon de maïs. A titre d'exemple, la matière première cosmétique Alcocap^{®} 300 commercialisée par la société NOURYON CHEMICALS, ou encore la matière première Nyuka^{®} commercialisée par la société NIPPON STARCH CHEMICAL CO. LTD, ou encore la matière première C*EmTex 12688 commercialisée par la société CARGILL INC., peut être utilisée comme source de sodium starch octenylsuccinate.

Selon un mode de réalisation particulier, le rapport pondéral entre l'hydroxypropyl starch phosphate et le sodium starch octenylsuccinate (INCI) est compris entre 1/15 et 1/1, avantageusement entre 1/10 et 2/3.

Selon un autre mode de réalisation particulier, le rapport pondéral entre l'hydroxypropyl distarch phosphate et le sodium starch octenylsuccinate (INCI) est compris entre 1/15 et 1/1, avantageusement entre 1/10 et 2/3.

A titre d'exemple, la matière première STARDESIGN^{™} POWER, commercialisée par la société CARGILL, et répondant aux désignations INCI sodium starch octenylsuccinate & hydroxypropyl starch phosphate peut être mise en oeuvre dans les compositions selon l'invention.

Selon un mode de réalisation particulier, l'hydroxypropyl starch phosphate (INCI) et le sodium starch octenylsuccinate (INCI) représentent entre 0,5% et 15% en poids total de la composition, avantageusement entre 1% et 10%, de préférence entre 3 et 6%.

Selon un autre mode de réalisation particulier, l'hydroxypropyl distarch phosphate (INCI) et le sodium starch octenylsuccinate (INCI) représentent entre 0,5% et 15% en poids total de la composition, avantageusement entre 1% et 10%, de préférence entre 3 et 6%.

L'association de l'hydroxypropyl starch phosphate (INCI), ou de l'hydroxypropyl distarch phosphate (INCI), et de sodium starch octenylsuccinate (INCI) avec au moins un filtre solaire dérivé de triazine permet d'obtenir des compositions solaires à haute protection, caractérisées par une innocuité cutanée et une résistance à l'eau accrues.

Par « résistance à l'eau » au sens de l'invention, on désigne la résistance de la composition à la mouillabilité, qui est la tendance d'une composition à absorber l'eau externe. En d'autres termes, il s'agit d'une meilleure résistance à l'élimination par rinçage de la composition, notamment des filtres solaires contenus dans ladite composition.

Des méthodes pour mesurer la résistance à l'eau sont connues à l'homme du métier.

Notamment, un indicateur classique de résistance à l'eau d'une composition cosmétique peut être obtenu en utilisant la méthode dite de l'angle de contact. Une goutte d'un liquide déposée sur la surface plane d'un corps solide forme un angle de contact θ à l'interface entre le liquide et le substrat, par exemple, une composition cosmétique. La valeur d'équilibre de cet angle se calcule par la loi de Young-Dupré, qui met en jeu les 3 valeurs de tension interfaciale existant pour un tel système, à savoir solide-liquide, liquide-gaz et enfin solide-gaz.

Une composition cosmétique est considérée comme étant résistante à l'eau selon cette méthode si l'angle de contact est supérieur à 35°.

Le paramètre de résistance à l'eau d'une composition solaire, telle que la composition selon l'invention, peut également être évalué de façon indirecte en mesurant le SPF conféré avant et après lavage à l'eau. Cette évaluation permet donc de déterminer la capacité de la composition, notamment des filtres solaires dérivés de triazine, à ne pas être éliminé par rinçage.

Selon un mode de réalisation particulier, la composition comprend un mélange constitué d'hydroxypropyl starch phosphate (INCI) et de sodium starch octenylsuccinate (INCI).

Selon un autre mode de réalisation particulier, la composition comprend un mélange constitué d'hydroxypropyl distarch phosphate (INCI) et de sodium starch octenylsuccinate (INCI).

Au sens de l'invention, par « filtre solaire dérivé de triazine », on désigne une molécule capable de filtrer les UV-A et/ou les UV-B et comprenant au moins un hétérocycle aromatique contenant trois atomes d'azote. L'isomère de triazine peut donc correspondre à une 1,2,4-triazine, une 1,3,5-triazine ou une 1,2,3-triazine.

Selon un mode de réalisation particulier de l'invention, le au moins un filtre solaire dérivé de triazine est choisi dans le groupe comprenant les 1,2,4-triazines, les 1,3,5-triazines et les 1,2,3-triazines.

Avantageusement, le au moins un filtre solaire dérivé de triazine est choisi dans le groupe comprenant les composés correspondant aux désignations INCI suivantes : bis ethylhexyloxyphenol methoxyphenyl triazine, diethylhexyl butamido triazone, ethylhexyl triazone, tris-biphenyl triazine, phenylene bis-diphenyltriazine et ethylkexyl bis-isopentylbenzoxazolylphenyl melamine.

Des filtres solaires dérivés de triazine aptes à être mis en oeuvre dans une composition selon l'invention sont disponibles sur le marché auprès de plusieurs fournisseurs. A titre d'exemple, les matières premières suivantes peuvent être mises en oeuvre dans la composition selon l'invention :
- le TINOSORB^{®} S/TINOSORB^{®} AQUA commercialisé par la société BASF et correspondant à la désignation INCI bis ethylhexyloxyphenol methoxyphenyl triazine (numéro CAS : 187393-00-6) ;
- l' UVASORB^{®} HEB commercialisé par la société SIGMA 3V et correspondant à la désignation INCI diethylhexyl butamido triazone (numéro CAS 154702-15-5) ;
- le TINOSORB^{®} A2B commercialisé par la société BASF et correspondant à la désignation INCI tris-biphenyl triazine (no CAS : 31274-51-8) ;
- l'UVINUL^{®} T150 commercialisé par la société BASF et correspondant à la désignation INCI ethylhexyl triazone (numéro CAS : 88122-99-0) ;
- le TRIASORB^{®} commercialisé par la société PLANTES & INDUSTRIE et correspondant à la désignation INCI phenylene bis-diphenyltriazine (numéro CAS 55514-22-2) ;
- l'UVASORB^{®} K2A commercialisé par la société SIGMA 3V et correspondant à la désignation INCI ethylhexyl bis-isopentylbenzoxazolylphenyl melamine (numéro CAS 288254-16-0) ;
- les dérivés de 1,3,5-triazine correspondant aux numéros CAS 2174063-28-4, 2174063-29-5 et 2174063-30-8, décrits dans le document EP 3 275 872 ;
- la 2,4,6-tris(4'-amino benzalmalonate de diéthyle)-s-triazine, la 2,4,6-tris(4'-amino benzalmalonate de diisopropyle)-1,3,5-triazine, la 2,4,6-tris(4'-amino benzalmalonate de diméthyle)-1,3,5,triazine ou encore la 2,4,6-tris(a-cyano-4-aminocinnamate d'éthyle)-1,3,5-triazine. Ces molécules sont décrites dans le document EP 0 507 692.

Selon un mode de réalisation particulier, la composition selon l'invention comprend au moins deux filtres solaires dérivés de triazine différents, voire au moins trois filtres solaires dérivés de triazine différents.

De préférence, la composition selon l'invention comprend au moins les filtres solaires dérivés de triazine correspondant aux désignations INCI suivantes : bis-ethylhexyloxyphenol methoxyphenyl triazine et diethylhexyl butamido triazone, avantageusement bis-ethylhexyloxyphenol methoxyphenyl triazine, diethylhexyl butamido triazone et ethylhexyl triazone.

Selon un mode de réalisation particulier, le au moins un filtre solaire dérivé de triazine représente entre 2% et 50% en poids total de la composition, avantageusement entre 5% et 40%, de préférence entre 10% et 30%.

Selon un mode de réalisation particulier, le au moins un filtre solaire dérivé de triazine représente plus de 10% en poids total de la composition, avantageusement plus de 12%, de préférence plus de 15%.

Il ressort de ce qui précède que le au moins un filtre solaire dérivé de triazine peut représenter entre 12% et 50% en poids de la composition, ou encore entre 15% et 50%.

Selon un autre mode de réalisation, outre le au moins un filtre solaire dérivé de triazine, la composition selon l'invention comprend au moins un filtre UV-A et/ou UV-B, organique et/ou minéral, qui peut se présenter en phase aqueuse (hydrophile) et/ou huileuse (lipophile).

Selon un mode de réalisation particulier, la composition selon l'invention comprend au moins un filtre UV-A, susceptible d'assurer une filtration complète de la partie nuisible du spectre solaire.

Selon un mode de réalisation particulier, la composition selon l'invention comprend au moins un filtre solaire UV-A choisi dans le groupe comprenant les composés correspondant aux désignations INCI suivantes : butyl methoxydibenzoylmethane, diethylamino hydroxybenzoyl hexyl benzoate, bis-(diethylaminohydroxybenzoyl benzoyl) piperazine, disodium phenyl dibenzimidazole tetrasulfonate et leurs mélanges.

A titre d'exemple, les filtres UV-A selon l'invention correspondent à la matière première Parsol 1789^{®} (INCI : butyl methoxydibenzoylmethane)commercialisée par la société DSM, UVINUL^{®} A+ (INCI : diethylamino hydroxybenzoyl hexyl benzoate) commercialisée par la société BASF ou encore la matière première C1332^{®} (INCI : bis-(diethylamino-hydroxybenzoyl benzoyl) piperazine ; numéro CAS 919803-06-8) commercialisée par la société BASF.

Avantageusement, la composition selon l'invention comprend un filtre UV-A correspondant à la désignation INCI disodium phenyl dibenzimidazole tetrasulfonate, notamment disponible sous la dénomination Neo Heliopan^{®} AP et commercialisé par la société SYMRISE.

Selon un mode de réalisation particulier, la composition selon l'invention comprend des écrans minéraux (ou filtres minéraux inorganiques), qui correspondent à des oxydes métalliques et/ou d'autres composés difficilement solubles ou insolubles dans l'eau, en particulier les oxydes de titane (TiO2), de zinc (ZnO), de fer (Fe₂O₃), de zirconium (ZrO₂), de silicium (SiO₂), de manganèse (par exemple MnO), d'aluminium (Al₂O₃), ou de cérium (Ce₂O₃), ou encore le trioxyde de bismuth (Bi₂O₃).

Avantageusement, les filtres minéraux inorganiques peuvent être utilisés sous forme de prédispersion huileuse ou aqueuse disponible sur le marché. Ces prédispersions peuvent être additionnées avantageusement d'auxiliaires de dispersion et/ou de médiateurs de solubilisation.

Les filtres minéraux inorganiques peuvent également être traités en surface ou encapsulés, afin de leur conférer un caractère hydrophile, amphiphile ou hydrophobe. Ce traitement de surface peut consister en ce que les filtres minéraux soient dotés d'une mince pellicule inorganique et/ou organique hydrophile et/ou hydrophobe.

Selon un mode de réalisation particulier, la composition selon l'invention comprend au moins un écran minéral choisi dans le groupe comprenant les composés correspondant aux désignations INCI suivantes : zinc oxide, titanium dioxide et leurs mélanges.

A titre d'exemple, le zinc oxide correspond à la matière première Z-COTE^{®} LSA et le titanium dioxide à la matière première T-Lite^{®}, commercialisées par la société BASF.

Les listes des filtres UV cités pouvant être mis en oeuvre au sens de la présente invention sont bien entendu donnés à titre indicatif et non limitatif.

De manière avantageuse, le au moins un filtre solaire et/ou écran minéral, à l'exception du ou des filtre(s) solaire(s) dérivé(s) de triazine, représente entre 0,1 et 30 % en poids total de la composition selon l'invention, avantageusement entre 0,5 et 20 %, encore plus avantageusement entre 1 et 15%.

La composition selon l'invention est exempte d'émulsionnant.

L 'absence d'émulsionnant peut également avoir un impact positif sur la résistance à l'eau des compositions.

Selon un mode de réalisation particulier, la composition selon l'invention est exempte des filtres solaires correspondant aux désignations INCI suivantes : 4-methylbenzylidene camphor, benzophenone-2, benzophenone-3, ethylhexyl methoxycinnamate et octocrylene.

Selon un mode de réalisation préféré, la composition selon l'invention comprend au moins les filtres correspondant aux désignations INCI suivantes : butyl methoxydibenzoylmethane, diethylamino hydroxybenzoyl hexyl benzoate, bis-ethylhexyloxyphenol methoxyphenyl triazine, diethylhexyl butamido triazone.

Selon un mode de réalisation particulier, la composition selon l'invention comprend au moins un solubilisant choisi dans le groupe comprenant les composés correspondant aux désignations INCI suivantes : dibutyl adipate, dicaprylyl carbonate, diisopropyl sebacate, caprylyl caprylate/caprate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate caprylic/capric triglicerides, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl maolnate, phenoxyethyl caprylate, isodecyl salicylate, dimethyl capramide et phenethyl benzoate.

Au sens de l'invention, par « solubilisant », on désigne un composé qui permet de solubiliser, disperser et/ou dissoudre au moins un filtre solaire dérivé de triazine de manière efficace et durable, c'est-à-dire en le stabilisant sous sa forme solubilisée et en empêchant ou réduisant sa recristallisation ou précipitation en formulation pendant toute la durée d'utilisation du produit (généralement, le produit doit être stable au moins 1 an après son ouverture).

Selon un mode de réalisation particulier, la composition selon l'invention comprend en outre au moins deux solubilisants choisis dans le groupe comprenant les composés correspondant aux désignations INCI suivantes : dibutyl adipate, dicaprylyl carbonate, diisopropyl sebacate, caprylyl caprylate/caprate, dicaprylyl ether, coco-caprylate, C12-15 alkyl benzoate, propylheptyl caprylate, butylène glycol dicaprylate/dicaprate,dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate caprylic/capric triglicerides, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl maolnate, phenoxyethyl caprylate, isodecyl salicylate, dimethyl capramide et phenethyl benzoate

Selon un mode de réalisation particulier, la composition selon l'invention comprend au moins les solubilisants suivants correspondant aux désignations INCI: dibutyl adipate, dicaprylyl carbonate et diisopropyl sebacate.

Selon un mode de réalisation particulier, les solubilisants représentent entre 5% et 80% en poids total de la composition, avantageusement entre 10% et 70%, de préférence entre 15% et 60%.

Des solubilisants aptes à être mis en oeuvre dans une composition selon l'invention sont disponibles sur le marché auprès de plusieurs fournisseurs. A titre d'exemple, les matières premières suivantes peuvent être mises en oeuvre dans la composition selon l'invention :
- plusieurs matières premières de la gamme CETIOL^{™} commercialisées par la société BASF, notamment le CETIOL^{™} RLF, CETIOL^{™} B, CETIOL^{™} CC, CETIOL^{™} O, CETIOL^{™} C5, CETIOL^{™} AB, CETIOL^{™} SENSOFT correspondant respectivement aux désignations INCI caprylyl caprylate/caprate, dibutyl adipate, dicaprylyl carbonate, dicaprylyl ether, coco-caprylate, C12-15 alkyl benzoate, propylheptyl caprylate ;
- le DUB^{™} DIS, DEA, DIBA, ZENOAT commercialisés par la société STEARINE DUBOIS correspondant respectivement aux désignations INCI diisopropyl sebacate, diethyl adipate, diisobutyl adipate et propanediol dicaprylate ;
- le MIGLYOL^{™} 8810 et T-C7 commercialisés par la société IOI Oleo GmbH correspondant respectivement aux désignations INCI butylène glycol dicaprylate/dicaprate e triheptanoin ;
- le Lexsolv^{™} A commercialisé par la société INOLEX correspondant aux désignations INCI dipropylene glycol dibenzoate et neopentyl glycol diheptanoate ;
- les COSMACOL^{™} ELI, ETI, ESI et LL commercialisés par la société SASOL et correspondant respectivement aux désignations INCI C12-13 alkyl lactate, C12-13 alkyl tartrate, tridecyl salicylate et lauryl lactate ;
- le Ceraphyl^{™} 41 ester commercialisé par la société ASHLAND et correspondant aux désignations INCI C12-C15 alkyl lactate ;
- les Finsolv^{™} EB et PG 22 commercialisés par la société INNOSPEC et correspondant respectivement aux désignations INCI, ethylhexyl benzoate et dipropylene glycol dibenzoate ;
- les CRODAMOL^{™} DA, DOA, OSU et PC commercialisés par la société CRODA et correspondant respectivement aux désignations INCI diisopropyl adipate, diethylhexyl adipate, diethylhexyl succinate et propylene glycol dicaprylate/dicaprate ;
- le ELDEW^{™} SL-205 commercialisé par la société AJINOMOTO et correspondant à la désignation INCI isopropyl lauroyl sarcosinate ;
- le Lexfeel^{™} Shine commercialisé par la société INOLEX et correspondant à la désignation INCI propylene glycol dibenzoate ;
- le TEGOSOFT^{™} XC et CT commercialisés par la société EVONIK et correspondant à la designtion INCI phenoxyethyl caprylate et caprylic/capric triglicerides ;
- le RONACARE^{™} AP commercialisé par la société MERCK KGaA et correspondant à la désignation INCI hydroxyl dimethoxybenzyl malonate ;
- le DERMOL^{™} IDSA commercialisé par la société Alzo INTL et correspondant à la désignation INCI isodecyl salicylate ;
- le Spectrasolv^{™} DMDA commercialisé par la société Hallstar et correspondant à la désignation INCU dimethyl capramide ;
- le X-TEND^{™} 226 commercialisé par la société Ashland et correspondant à la désignation INCI phenethyl benzoate.

Selon un mode de réalisation particulier, la composition selon l'invention présente un SPF égal ou supérieur à 30, avantageusement égal ou supérieur à 50, de préférence égal ou supérieur à 70, voire égal ou supérieur à 100.

Selon un mode de réalisation préféré, la composition selon l'invention comporte un ratio de protection UV-A/UV-B égal ou supérieur à 1/3.

Avantageusement, la composition selon l'invention présente une longueur d'onde critique (λc) supérieure à 370 nm. Cette valeur, qui est déterminée par des méthodes in vitro connues de l'homme du métier, correspond à la longueur d'onde pour laquelle l'intégrale de la courbe du spectre d'absorption commençant à 290 nm atteint 90 % de l'intégrale entre 290 et 400 nm.

Selon un mode de réalisation particulier, la composition selon l'invention peut en outre comprendre un « booster » de SPF, c'est-à-dire un agent amplificateur du facteur de protection solaire, et/ou un photostabilisant, c'est à dire un ingrédient qui permet d'augmenter le SPF ou de photostabiliser les filtres, un tel ingrédient n'étant pas considéré lui-même comme un filtre solaire. On peut par exemple citer :
- le butyloctyl salicylate (INCI), photostabilisant représentant avantageusement entre 0,01% et 10% en poids total de la composition, encore plus avantageusement ente 0,1% et 2%. Cette matière première est, par exemple, commercialisée par la société HALLSTAR sous le nom de Hallbrite^{®} BHB;
- le benzotriazolyl dodecyl p-cresol (INCI), photostabilisant représentant avantageusement entre 0,01% et 10% en poids total de la composition, encore plus avantageusement entre 0,1% et 2%. Cette matière première est, par exemple, commercialisée par la société BASF sous le nom de TINOGARD^{®} TL ;
- le pongamol (INCI), molécule végétale absorbant dans les UV-A, représentant avantageusement entre 0,5 et 2% en poids total de la composition, encore plus avantageusement de l'ordre de 1%. A titre d'exemple, la matière première Pongamia Extract commercialisée par la société GIVAUDAN peut être utilisée dans le cadre la présente invention ;
- l'ethylhexyl methoxycrylene (INCI), photostabilisant, solubilisant et « booster » de SPF représentant avantageusement entre 1% et 5% en poids total de la composition. La matière première SolaStay^{®} S1 commercialisée par la société HALLSTAR peut être utilisée dans le cadre de la présente invention ;
- un copolymère de styrène acrylate (INCI : styrene/acrylate copolymer), représentant de préférence entre 1% et 10% en poids total de la composition selon l'invention. Les matières premières SunSpheres^{®} H53 et SunSpheres^{®} PGL Polymer, commercialisées par la société DOW CHEMICALS, peuvent être utilisées dans le cadre de la présente invention ;
- le diethylhexyl syringylidene malonate (INCI), représentant avantageusement entre 1% et 10% en poids total de la composition. La matière première OXYNET^{®} ST, commercialisée par la société MERCK, peut être utilisée dans le cadre de la présente invention ;
- un polyester hydrodispersible, correspondant aux désignations INCI polyester-5 (and) Sodium silicoaluminate, représentant avantageusement entre 1% et 10% en poids total de la composition, notamment l'EASTMANN AQTM38S Polymer commercialisé par la société SAFIC-ALCAN ;
- un copolymère d'acrylate ayant une température de transition vitreuse de -5°C à -15°C telle que mesurée par calorimétrie différentielle à balayage, ledit copolymère représentant avantageusement entre 1% et 10% en poids total de la composition. Par exemple, un polymère correspondant à la désignation INCI Acrylate copolymer, tel que la matière première EPITEX 66, commercialisée par la société DOW CHEMICALS, peut être utilisée dans le cadre de la présente invention.

Selon un mode de réalisation particulier, la phase grasse de la composition selon l'invention représente entre -30% et 60%.

Au sens de l'invention, par « phase grasse » on désigne l'ensemble des ingrédients constitué par les filtres solaires dérivés de triazine, les filtres solaires lipophiles autres que les filtres solaires dérivés de triazine, les solubilisants, les actifs lipophiles et les excipients lipophiles.

La phase aqueuse de la composition selon l'invention représente entre 30% et 80% en poids total de la composition de phase grasse, avantageusement ente 40% et 70%.

Selon un autre mode de réalisation, la composition selon l'invention peut comprendre, en outre, un extrait de la bactérie *Arthrobacter agilis*, notamment un extrait riche en caroténoïdes, tel que décrit dans le document WO 2014/167247. Avantageusement, la composition selon l'invention comprend de 0,00001% à 0,1% en poids total de la composition, encore plus avantageusement de 0,0001% à 0,001 % d'un tel extrait sec.

Dans un mode de réalisation privilégié, la composition selon l'invention comprend, en outre, d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une préparation selon l'invention contre le stress oxydatif ou à l'encontre de l'effet des radicaux libres peut être encore améliorée si celle-ci comprend, en outre, un ou plusieurs antioxydants, aisément sélectionnés par l'homme du métier par exemple dans la liste suivante : le totarol, le magnolol, l'honokiol, les acides aminés et leurs dérivés, des peptides (D et/ou L-carnosine) et leurs dérivés (par exemple l'ansérine, l'hypotaurine, la taurine), les caroténoïdes, les carotènes (α-carotène, β-carotène, lycopène) et leurs dérivés, l'acide chlorogénique et ses dérivés, l'acide lipoïque et ses dérivés (acide dihydrolipoïque), l'aurothioglucose, le propylthiouracile et autres thiols (thiorédoxine, glutathion, cystéine, cystine, cystamine et leurs esters glycosyle, N-acétyle, méthyle, éthyle, propyle, amyle, butyle et lauryle, palmitoyle, oléyle, γ-linoléique, cholestéryle et glycéryle) ainsi que des sels de ceux-ci, le thiodipropionate de dilauryle, le thiodipropionate de distéaryle, l'acide thiodipropionique et ses dérivés, les composés sulfoximine (sulfoximine de buthionine, l'homocystéine sulfoximine, les buthionine sulfones, penta-, hexa- et heptathionine sulfoximine), des agents chélatants (tels que les acides α-hydroxygras, l'acide palmitique, l'acide phytique, la lactoferrine), les α-hydroxyacides (tels que l'acide citrique, lactique, ou malique), l'acide humique, l'acide biliaire, les extraits de bile, la bilirubine, la biliverdine, le tétraméthylène phosphonate pentasodique d'éthylènediamine et ses dérivés, les acides gras insaturés et leurs dérivés, la vitamine A et ses dérivés (palmitate de vitamine A), le benzoate de coniféryle de la résine de benjoin, l'acide rutinique et ses dérivés, l'α-glycosyl rutine, l'acide férulique et ses dérivés, le furfurylideneglucitol, la carnosine, le butylhydroxytoluène, le butylhydroxyanisole, l'acide nordihydroguaiarétique, trihydroxybutyrophenone, la quercétine, l'acide urique et ses dérivés, le mannose et les dérivés de ceux-ci, le zinc et ses dérivés (ZnO, ZnSO4), le sélénium et ses dérivés (sélénométhionine), les stilbènes et leurs dérivés (l'oxyde de stilbène, l'oxyde trans-stilbène).

Dans un mode de réalisation particulier, la composition selon l'invention comprend, en outre, de l'acide glycyrrhétinique, un dérivé ou un sel de cet acide, ou encore l'acetyl dipeptide-1 cetyl ester (INCI) utilisés comme agents anti-inflammatoires et représentant globalement entre 0,01% et 2 % en poids total de la composition, de préférence entre 0,1% et 1%.

Selon une autre caractéristique privilégiée de l'invention, la composition cosmétique et/ou dermatologique comprend au moins un, voire tous les constituants suivants exerçant une activité biologique *in vivo* sur les cellules de la peau, des lèvres, des cheveux et/ou des muqueuses soumises à un rayonnement UV-A et/ou UV-B, respectivement :
- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre 0,001 et 10% en poids total de la composition, encore plus avantageusement entre 0,02 et 2%, de préférence de l'ordre de 0,04%;
- un agent limitant l'immunosuppression, tel que par exemple la vitamine PP, représentant avantageusement entre 0,001 et 1% en poids total de la composition, encore plus avantageusement de 0,01% à 0,3%;
- un agent protecteur de la protéine p53, tel que par exemple l'épigallocatéchine gallate (EGCG), représentant avantageusement entre 0,001 et 0,1% en poids total de la composition, encore plus avantageusement de 0,005% à 0,05%.

La composition selon l'invention peut également comprendre, en outre, des extraits peptidiques de soja et/ou de blé, tels que ceux décrits dans le document EP 2 059 230.

En pratique, les extraits peptidiques proviennent de graines de soja et de blé sont issus d'une hydrolyse enzymatique desdites graines par l'intermédiaire de peptidases qui permettent de récupérer des peptides d'une taille moyenne de 700 Daltons. Préférentiellement, l'extrait peptidique de soja est l'extrait identifié sous le numéro CAS 68607-88-5 de même que l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et/ou de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentant de 0,01 à 20 % en poids total de la composition, avantageusement de 0,1% à 10 %, encore plus avantageusement de 0,2% à 0,7%.

Dans un mode de réalisation alternatif, la composition selon l'invention comprend, conformément aux enseignements du document FR 2 865 398, l'association d'au moins un acide aminé choisi dans le groupe constitué par l'ectoïne, la créatine, l'ergothionéine et/ou la carnosine, ou leurs sels physiologiquement acceptables, et du mannitol ou un dérivé du mannitol.

De préférence, la composition selon l'invention comprend dans un milieu physiologiquement acceptable l'acide aminé ou l'un de ses sels, seul ou en mélange dans des proportions comprises entre 0,001% et 10 % en poids total de la composition, et de préférence entre 0,01% et 5 %.

La composition selon la présente invention comprend, de préférence, dans un milieu physiologiquement acceptable, du mannitol ou l'un de ses dérivés, dans des proportions comprises entre 0,01% et 30 % en poids total de la composition, avantageusement entre 0,1% et 10 %.

Dans un mode de réalisation privilégié, la composition selon l'invention comprend de l'ectoïne et du mannitol.

Selon un mode de réalisation particulier, la composition selon l'invention comprend un ou plusieurs autres agents bronzants ou autobronzants. Il peut s'agir d'un autobronzant qui réagit avec les acides aminés de la peau selon une réaction de Maillard ou par l'intermédiaire d'une addition de Michael, ou bien un promoteur de la mélanogénèse ou un composé propigmentant qui favorise le bronzage naturel de la peau. Une telle substance est de préférence présente dans la composition en quantité allant de 0,01% à 20% en poids total de la composition, avantageusement de 0,5% à 15%, encore plus avantageusement de 1% à 8%.

Les substances autobronzantes peuvent être le 1,3-dihydroxyacétone (DHA), le glycérolaldéhyde, l'hydroxyméthylglyoxal, l'γ-dialdéhyde, l'érythrulose, le 6-aldo-D-fructose, la ninhydrine, la 5-hydroxy-1,4-naphtoquinone (juglone), la 2-hydroxy-1,4-naphtoquinone (lawsone), ou leur combinaison.

Les substances propigmentantes peuvent être l'hormone stimulant les mélanocytes (α-MSH), des analogues peptidiques de l'α-MSH, des agonistes du récepteur à l'endothéline-1, des agonistes des récepteurs µ opiacés, des agents stimulateurs d'AMPc, des agents stimulateurs de tyrosinase.

Dans un mode de réalisation particulier, la composition selon l'invention comprend, en outre, en qualité d'autobronzant, une combinaison de dihydroxy methylchromonyl palmitate et/ou le dimethylmethoxy chromanol ainsi qu'une forme lipophile de la tyrosine.

Cette association de principes actifs permet de stimuler efficacement le bronzage. Le dihydroxy méthylchromonyl palmitate (numéro CAS : 1387636-35-2) correspond, par exemple, à l'ingrédient cosmétique commercialisé par la société MERCK sous le nom de RonaCare^{®} Bronzyl. Le dimethylmethoxy chromanol (numéro CAS : 83923-51-7) correspond, par exemple, à l'ingrédient cosmétique commercialisé par la société LIPOTEC SA sous le nom de lipochromone-6.

Selon un mode de réalisation particulier, le dihydroxy méthylchromonyl palmitate ou le dimethylmethoxy chromanol est compris dans la composition selon l'invention à hauteur de 0,01% à 10% en poids total de la composition, avantageusement de 0,05% à 10%, encore plus avantageusement de 0,1% à 5%, plus particulièrement de 0,1% à 0,5%.

La forme lipophile de la tyrosine, au sens de l'invention, est un ingrédient à base de tyrosine et présente un caractère lipophile plus prononcé que la tyrosine. La forme lipophile de la tyrosine peut notamment correspondre à l'oléoyl tyrosine (Numéro CAS : 147732-57-8), qui se retrouve, par exemple, dans l'ingrédient cosmétique liquide TYR-OL, commercialisé par la société SEDERMA, et qui comprend environ 50% en poids d'oléoyl tyrosine dans du butylène glycol (environ 30% + environ 20% d'acide oléique), ou bien dans l'ingrédient cosmétique liquide TYR-EXCEL, commercialisé par la société SEDERMA, qui comprend environ 50% en poids d'oléoyl tyrosine, environ 20% en poids d'acide oléique (N° CAS : 112-80-1) et environ 30% en poids d'huile de *Luffa cylindrica* (huile de pépins de courge éponge; N° CAS : 1242417-48-6).

Selon un autre mode de réalisation, la forme lipophile de la tyrosine correspond à une huile végétale dans laquelle a été formulée la tyrosine.

Selon un mode de réalisation particulier, l'huile végétale est de l'huile de tournesol oléique, avantageusement désodorisée. Ainsi, la matière première OLEOACTIF TYROSINE BASE HELIANTHUS ANNUS commercialisée par l'entreprise OLEOS, et correspondant aux désignations INCI *Helianthus annuus* seed oil (and) tyrosine (and) glyceryl stéarate, peut être utilisée dans le cadre de la présente invention.

En pratique, la forme lipophile de la tyrosine, telle que dans les ingrédients cosmétiques à base d'oléoyl-tyrosine (avantageusement à 50% en poids) ou de la tyrosine formulée dans de l'huile végétale, représente entre 0,1% et 10% en poids total de la composition, avantageusement entre 1 et 3%, encore plus avantageusement entre 1% et 1,5%.

La composition selon l'invention peut également comprendre des actifs ayant des propriétés dépigmentantes, comme par exemple :
- de l'azéilate de lysine, ou d'autres dérivés ou sels de l'acide azélaïque ;
- de l'andrographolide, notamment l'extrait d'*Andrographis paniculata* correspondant à la désignation INCI *Andrographis paniculata* leaf extract ;
- de l'acide ascorbique natif (vitamine C) ou ses dérivés, notamment les dérivés correspondant aux INCI Ascorbyl Glucoside, Ethyl ascorbic acid, Ascorbyl methylsilanol pectinate, Sodium ascorbyl phosphate et Ascorbyl tetraisopalmitate, avantageusement l'ascorbyl glucoside ;
- de l'arbutine ou un extrait végétal la contenant, notamment l'extrait de busserole correspondant à la désignation INCI *Arctostaphylos uva-ursi* leaf extract ;
- de la glabridine ou un extrait végétal la contenant, notamment les extraits de réglisse correspondant à la désignation INCI *Glycyrrhiza glabra* root extract, *Glycyrrhiza inflata* root extract, *Glycyrrhiza uralensis* root extract ;
- les peptides biomimétiques correspondant aux désignations INCI hexapeptide 2 et/ou nonapeptide-1 ;
- un extrait aqueux d'une algue dénommée *Palmaria palmata,* notamment l'extrait correspondant à la désignation INCI *Palmaria palmata* extract ;
- le 4-n-butylresorcinol ;
- de la vitamine PP, également appelée niacinamide ou nicotinamide, et ses dérivés;
- ou leurs mélanges.

La composition selon l'invention peut également comprendre des actifs ayant des propriétés cicatrisantes ou régénérantes comme par exemple un agent antimicrobien choisi parmi les actifs correspondant aux désignations INCI suivantes : copper sulfate, zinc sulfate, sodium hyaluronate, *Vitis vinifera* (grape) vine extract et leurs mélanges ; un extrait de la plante *Centella asiatica,* ou les terpènes de *C. asiatica* asiaticoside, acide madecassique et acide asiatique, ainsi que leurs mélanges.

La composition selon l'invention peut également comprendre des actifs ayant des propriétés sébocorrectices, kératolytiques, séboregulatrices et/ou une activité antiacnéique, afin de permettre la formulation de produits solaires traitant l'acné.

Par exemple, la composition selon l'invention peut comprendre un agent antimicrobien choisi parmi les actifs correspondant aux désignations INCI propyl gallate, dodecyl gallate, *Ginkgo biloba* leaf extract, bakuchiol, dihydromyricetine, zinc gluconate, salicylic acid et leurs mélanges.

La composition selon l'invention peut en outre comprendre au moins un ingrédient choisi dans la liste suivante :
- un agent apte à filtrer la lumière visible, en particulier la lumière bleue ;
- un extrait des algues *Laminaria ochroleuca, Blidingia minima* ou *Laminaria saccharina* ;
- un extrait de la plante *Zanthoxylum alatum* ;
- du panthénol ;
- un extrait de bois de cade ;
- un extrait de Boldo ;
- un extrait de Reine des prés ;
- un extrait d'huile de karanja issue du *Pongamia glabra* ;
- des paraffines linéaires ;
- de l'ATP (adénosine-5 tri-phosphate), du Gp4G (diguanosine tétraphosphate) ou de l'Ap4A (diadénosine tétraphosphate) ; ou
- un acide aminé choisi dans le groupe constitué de la décarboxycarnosine, la glutamine et leurs sels.

La composition selon l'invention peut également comprendre des adjuvants comme ceux habituellement utilisés dans le domaine de la cosmétique, tels que des conservateurs, des antioxydants, des agents complexants, des solvants, des parfums, des charges, des bactéricides, des électrolytes, des absorbeurs d'odeur, des matières colorantes ou encore des vésicules lipidiques. Le choix de ces adjuvants, ainsi que leurs concentrations, doivent être déterminés de telle sorte qu'ils ne modifient pas les propriétés et les avantages recherchés pour la composition de la présente invention.

La composition de l'invention se présente sous une forme adaptée à une administration, ou application, topique et plus particulièrement pour application sur la peau, les lèvres, les cheveux et/ou les muqueuses.

Ainsi et dans le cadre de l'invention, une telle composition est notamment destinée à la protection de la peau et/ou des phanères, en particulier les muqueuses, les lèvres et les cheveux, contre le rayonnement UV.

La composition de l'invention peut se présenter sous toutes les formes galéniques normalement utilisées dans les domaines cosmétique et dermatologique.

Selon un mode de réalisation particulier, la viscosité de la composition selon l'invention est comprise entre 60000 et 160000 mPa.s, avantageusement entre 80000 et 140000 mPa.s.

Selon l'invention, la viscosité est mesurée à température ambiante au moyen d'un viscosimètre Brookfield DV-II+ (conditions : pourcentage de lecture 83,5%, Mobile 6 Vitesse 6 (M6V6)).

D'autres moyens pour mesurer la viscosité des compositions cosmétiques sont connus à l'homme de l'art.

Selon un mode de réalisation particulier, la composition selon l'invention est un gel crème, avantageusement un gel crème stable.

La formulation galénique sous forme de gel crème est connue à l'homme de l'art. Il s'agit de la dispersion d'une phase lipidique interne liquide, solide ou semi-solide discontinue dans une solution aqueuse externe continue. Le gel crème est un système métastable et a par conséquent tendance à se convertir en un état avec deux phases discrètes intrinsèquement cohérentes. Dans une émulsion conventionnelle, le choix d'un émulsifiant approprié empêche la séparation de phase. Contrairement aux émulsions conventionnelles, le gel crème, avantageusement le gel crème stable, ne contient généralement que de petites quantités d'émulsionnant (jusqu'à 2% en poids maximum), voire en sont exemptes.

De préférence, la composition selon l'invention est un gel crème, avantageusement exempte d'émulsionnants.

Avantageusement, la composition selon l'invention est un gel crème stable, de préférence exempte d'émulsionnants.

Selon un autre mode de réalisation particulier, la composition selon l'invention se présente sous la forme d'une émulsion H/E, avantageusement stable, qui comprend moins de 2% en poids total de la composition d'émulsionnant, avantageusement moins de 1%, moins de 0,5% voire moins de 0,1%, de préférence exempte d'émulsionnant.

Au sens de la présente invention, par « composition stable », on désigne une composition présentant une dispersion homogène et durable de la phase grasse dans la phase aqueuse, sans séparation de phases, floculation et/ou formation d'agrégats.

Selon un mode de réalisation particulier, tout conservateur, autorisé ou non autorisé par la réglementation, (annexe V de la Directive européenne sur les produits cosmétiques), apte à être utilisé dans des compositions cosmétiques ou dermatologiques peut être mis en oeuvre dans la formulation d'une composition selon l'invention.

Selon un mode de réalisation particulier, les conservateurs utilisables dans les compositions selon l'invention sont des alcanediols.

De préférence, les conservateurs selon l'invention sont des 1,2-alcanediols ou 1,3-alcanediols.

Selon un mode de réalisation particulier, l'alcanediol est choisi dans le groupe comprenant les composés correspondants aux désignations INCI suivantes : propylene glycol, 1,3-propanediol, 2-methyl-1,3-propanediol, 1,2-pentanediol, 1,2-hexanediol, caprylyl glycol, 1,2-decanediol, 2-methyl-2,4-pentanediol et leurs mélanges.

Ces conservateurs sont disponibles auprès de plusieurs fournisseurs de matières premières cosmétiques.

A titre d'exemple, on peut citer :
- le EVO-100^{™} commercialisé par la société ARCHER DANIELS MIDLAND COMPANY et correspondant à la désignation INCI propylene glycol ;
- le ZEMEA^{™} commercialisé par la société DUPONT-TATE & LYLE BIOPRODUCTS et correspondant à la désignation INCI 1,2-propanediol ;
- le DUB DIOL^{™} commercialisé par la société STEARINERIE DUBOIS et correspondant à la désignations INCI 2-methyl-1,3-propanediol ;
- l'Hydrolite-5^{™}, Hydrolite-6^{™}, Hydrolite-8^{™} et le SymClariol 344028^{™} commercialisés par la société SYMRISE et correspondant respectivement aux désignations INCI 1,2-pentanediol, 1,2-hexanediol, caprylyl glycol et 1,2-decanediol ; ou encore
- l'Hexasol^{™} commercialisé par la société ARKEMA et correspondant à la désignation INCI hexylene glycol.

Selon un autre mode de réalisation, le au moins un agent conservateur est un ammonium quaternaire choisi dans le groupe comprenant le chlorure de behentrimonium, le bromure de cétrimonium, le bromure de myrtrimonium, le chlorure de cétrimonium, le bromure de laurtrimonium, le chlorure de laurtrimonium, le bromure de stéartrimonium, le chlorure de stéartrimonium, le chlorure de benzéthonium, le chlorure de benzalkonium et leurs mélanges.

Selon un mode de réalisation particulier, l'agent conservateur correspondant à un ammonium quaternaire est le bromure de cétrimonium et/ou le bromure de myrtrimonium, avantageusement le bromure de cétrimonium.

A titre d'exemple, le bromure de cétrimonium est commercialisé par la société MERCK sous la dénomination commerciale RONACARE^{™} CETRIMONIUM BROMIDE et le bromure de myrtrimonium est commercialisé par la société VERTELLUS PERFORMANCE MATERIALS sous la dénomination de Mytab.

D'autres conservateurs adaptés à une utilisation dans la composition comprenant le CSC et/ou CSL selon l'invention sont, par exemple :
- le potassium sorbate (INCI), le sodium benzoate (INCI) et le benzyl alcohol (INCI) commercialisés par la société AZELIS UK sous la dénomination Paratexin^{™} KS, Paratexin^{™} SBG et Paratexin^{™} BA, respectivement ;
- le p-anisic acid (INCI) et levulinic acid (INCI) commercialisés par la société COSPHATEC GMBH sous la dénomination Cosphaderm^{™} et Cophaderm^{™} LA-T, respectivement ;
- le dehydroacetic acid (INCI) et le polyaminoprypyl guanide (INCI), commercialisés par la société LONZA sous la dénomination Geogard^{™} 111A et Cosmocil^{™} CQ, respectivement ; ou encore
- le phenoxyethanol (INCI) commercialisé par la société CLARIANT INTERNATIONAL LTD sous la dénomination Phenoxetol^{™}.

Selon un mode de réalisation particulier, la composition selon l'invention comprend entre 0,001% et 5% en poids total de la composition d'agent conservateur, avantageusement entre 0,01% et 2%, de préférence entre 0,1% et 1%.

Avantageusement, la composition comprend également un ou plusieurs polymères épaississants, qui peuvent contribuer à stabiliser la composition. Les polymères épaississants selon l'invention peuvent être non ioniques, anioniques, amphotères ou cationiques. Les unités de base des polymères de l'invention peuvent être des mono- ou disaccharides.

On peut notamment citer comme polymères susceptibles d'être employés, les gommes natives suivantes, ainsi que leurs dérivés :
a) les exsudats d'arbres ou d'arbustes dont la gomme arabique, la gomme ghatti, la gomme karaya, la gomme tragacanthe et leurs dérivés;
b) les gommes issues d'algues dont l'agar, les alginates, les carraghénanes, les furcelleranes et leurs dérivés;
c) les gommes issues de semences ou tubercules dont la gomme de guar, la gomme de caroube, la gomme de fenugrec, la gomme de tamarin, la gomme de konjac et leurs dérivés;
d) les gommes microbiennes dont la gomme de xanthane, la gomme de gellane, la gomme de scléroglucane ou de sclerotium, et leurs dérivés;
e) les extraits de plantes dont la cellulose, l'inuline et leurs dérivés.

Avantageusement, les polymères épaississants utilisés dans les compositions selon l'invention sont la gomme de xanthane et/ou la gomme de sclerotium, avantageusement les deux gommes utilisées de façon simultanée. A titre d'exemple, la matière première cosmétique AMIGEL^{®} commercialisée par la société ALBAN MULLER et répondant à la désignation INCI sclerotium gum peut être utilisée comme source de scleroglucan ; tandis que la matière première KELTROL CG-SFT^{®} commercialisée par la société CP Kelco et répondant à la désignation INCI xanthan gum peut être utilisé comme source de gomme de xanthane.

Selon un mode de réalisation particulier, la composition selon l'invention comprend entre 0,01% et 6% en poids total de la composition de polymères épaississants avantageusement entre 0,1% et 3%.

Selon un autre aspect, l'invention concerne une composition telle que décrite précédemment pour utilisation pour la protection de la peau, les lèvres, les cheveux et/ou les muqueuses contre le rayonnement solaire ultraviolet.

Selon un mode de réalisation particulier, la composition selon l'invention est utilisée pour filtrer le rayonnement UV compris entre 280 et 400 nm, en particulier entre 300 et 380 nm.

La manière dont l'invention peut être réalisée et les avantages qui en découlent ressortiront mieux des exemples de réalisation qui suivent, donnés à titre indicatif et non limitatif.

### Exemples de réalisation de l'invention

Les pourcentages indiqués sont donnés en poids de produit par rapport au poids total de la composition dans les tableaux ci-dessous.

### Exemple I- gel crème SPF50+ selon l'invention

| [Tableaux 1] | |
|---|---|
| **Nom INCI** | **% INCI** |
| Aqua/water/eau | 35,239 |
| IDibutyl adipate | 18,00 |
| IDicaprylyl carbonate | 9,997 |
| Diethylheayl butamido triazone | 9,00 |
| IDiethylamino hydroxybenzoyl hexyl benzoate | 7,00 |
| Glycerin | 5,00 |
| Diisopropyl sebacate | 4,80 |
| Bis-ethylhexyloxyphenol methoxyphenyl triazine | 3,50 |
| Sodium starch octenylsuccinate | 3,20 |
| Butylene glycol | 1,00 |
| Phenoxyethanol | 1,00 |
| Sodium citrate | 1,00 |
| Hydroxypropyl starch phosphate | 0,80 |
| Sclerotium gum | 0,30 |
| Sodium hyaluronate | 0,10 |
| Citric acid | 0,061 |
| Tocopherol | 0,003 |

## Revendications

1. Composition solaire comprenant :
- une phase aqueuse ;
- une phase grasse ;
- de l'hydroxypropyl starch phosphate (INCI) ;
- du sodium starch octenylsuccinate (INCI) ;
- au moins un filtre solaire dérivé de triazine
la phase grasse représente entre 20% et 70% en poids total de la composition,
la composition est exempte d'émulsionnant,
caractérisée ce que le au moins un filtre solaire dérivé de triazine est le bis-ethylhexyloxyphenol methoxyphenyl triazine
ou
**caractérisée en ce que** le rapport pondéral entre l'hydroxypropyl starch phosphate (INCI) et le sodium starch octenylsuccinate (INCI) est compris entre 1/15 et 1/1, et **en ce que** l'hydroxypropyl starch phosphate (INCI) et le sodium starch octenylsuccinate (INCI) représentent entre 0.5% et 15% en poids total de la composition.

2. Composition selon l'une des revendications précédentes, **caractérisée en ce qu'**elle comprend au moins deux, avantageusement au moins trois, filtres solaires dérivés de triazine différents.

3. Composition selon l'une des revendications précédentes, **caractérisée en ce qu'**elle comprend au moins les filtres solaires dérivés de triazine correspondant aux désignations INCI suivantes : bis-ethylhexyloxyphenol methoxyphenyl triazine et diethylhexyl butamido triazone, avantageusement bis-ethylhexyloxyphenol methoxyphenyl triazine et diethylhexyl butamido triazone et ethylhexyl triazone.

4. Composition selon l'une des revendications précédentes, **caractérisée en ce que** le au moins un filtre solaire dérivé de triazine représente entre 2% et 50% en poids total de la composition.

5. Composition selon la revendication 4, **caractérisée en ce que** le au moins un filtre solaire dérivé de triazine représente plus de 10% en poids total de la composition, avantageusement plus de 12%, de préférence plus de 15%.

6. Composition selon l'une des revendications précédentes, **caractérisée en ce qu'**elle se présente sous la forme d'un gel crème, avantageusement un gel crème stable.

7. Composition selon l'une des revendications précédentes, **caractérisée en ce qu'**elle comprend en outre au moins deux solubilisants choisis dans le groupe comprenant les composés correspondant aux désignations INCI suivantes : dibutyl adipate, dicaprylyl carbonate, diisopropyl sebacate, caprylyl caprylate/caprate, dicaprylyl ether, coco-caprylate, C12-15 alkyl benzoate, propylheptyl caprylate, butylene glycol dicaprylate/dicaprate,dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate caprylic/capric triglicerides, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate.

8. Composition selon la revendication 7, **caractérisée en ce qu'**elle comprend au moins les solubilisants suivants correspondant aux désignations INCI: dibutyl adipate, dicaprylyl carbonate et diisopropyl sebacate.

9. Composition selon l'une des revendications précédentes, **caractérisée en ce que** les solubilisants représentent entre 5% et 80% en poids total de la composition, avantageusement entre 10% et 70%, de préférence entre 15% et 60%.

10. Composition selon l'une des revendications précédentes, **caractérisée en ce qu'**elle présente un Facteur de Protection Solaire (FPS) égal ou supérieur à 30, avantageusement égal ou supérieur a 50, de préférence égal ou supérieur à 70, voire égal ou supérieur à 100.

11. Composition telle que définie dans l'une des revendications 1 à 10 pour utilisation pour la protection de la peau, des levres, des cheveux et/ou des muqueuses contre le rayonnement solaire ultraviolet.

## Patentansprüche

1. Sonnenschutzmittelzusammensetzung, umfassend:
- eine wässrige Phase;
- eine Fettphase;
- Hydroxypropylstärkephosphat (INCI);
- Natriumstärkeoctenylsuccinat (INCI);
- mindestens einen von Triazin abgeleiteten Sonnenschutzfilter;
wobei
die Fettphase zwischen 20 % und 70 % des Gesamtgewichts der Zusammensetzung darstellt,
die Zusammensetzung frei von Emulgatoren ist,
**dadurch gekennzeichnet, dass** der mindestens eine von Triazin abgeleitete Sonnenschutzfilter Bis-Ethylhexyloxyphenol-Methoxyphenyl-Triazin ist
oder
**dadurch gekennzeichnet, dass** das Gewichtsverhältnis zwischen dem Hydroxypropylstärkephosphat (INCI) und dem Natriumstärkeoctenylsuccinat (INCI) zwischen 1/15 und 1/1 umfasst ist und dass das Hydroxypropylstärkephosphat (INCI) und das Natriumstärkeoctenylsuccinat (INCI) zwischen 0,5 % und 15 % des Gesamtgewichts der Zusammensetzung darstellen.

2. Zusammensetzung gemäß einem der vorstehenden Ansprüche, **dadurch gekennzeichnet, dass** sie mindestens zwei, vorteilhafterweise mindestens drei, verschiedene von Triazin abgeleitete Sonnenschutzfilter umfasst.

3. Zusammensetzung gemäß einem der vorstehenden Ansprüche, **dadurch gekennzeichnet, dass** sie mindestens die von Triazin abgeleiteten Sonnenschutzfilter umfasst, entsprechend den folgenden INCI-Bezeichnungen: Bis-Ethylhexyloxyphenol-Methoxyphenyl-Triazin und Diethylhexyl-Butamido-Triazon, vorzugsweise Bis-Ethylhexyloxyphenol-Methoxyphenyl-Triazin und Diethylhexyl-Butamido-Triazon sowie Ethylhexyl-Triazon.

4. Zusammensetzung gemäß einem der vorstehenden Ansprüche, **dadurch gekennzeichnet, dass** der mindestens eine von Triazin abgeleitete Sonnenschutzfilter zwischen 2 % und 50 % des Gesamtgewichts der Zusammensetzung darstellt.

5. Zusammensetzung nach Anspruch 4, **dadurch gekennzeichnet, dass** der mindestens eine von Triazin abgeleitete Sonnenschutzfilter mehr als 10 % des Gesamtgewichts der Zusammensetzung darstellt, bevorzugt mehr als 12 %, vorzugsweise mehr als 15 %.

6. Zusammensetzung gemäß einem der vorstehenden Ansprüche, **dadurch gekennzeichnet, dass** sie in der Form einer Gel-Creme vorliegt, bevorzugt einer stabilen Gel-Creme.

7. Zusammensetzung gemäß einem der vorstehenden Ansprüche, **dadurch gekennzeichnet, dass** sie außerdem mindestens zwei Lösungsvermittler umfasst, ausgewählt aus der Gruppe, die die Verbindungen umfasst, entsprechend den folgenden INCI-Bezeichnungen: Dibutyladipat, Dicaprylylcarbonat, Diisopropylsebacat, Caprylylcaprylat/caprat, Dicaprylylether, Kokos-Caprylat, C12-15-Alkylbenzoat, Propylheptylcaprylat, Butylenglykoldicaprylat/dicaprat, Dipropylenglykoldibenzoat, Neopentylglykoldiheptanoat, Triheptanoin, C12-13-Alkyllactat, Ethylhexylbenzoat, C12-C15-Alkyllactat, C12-13-Alkyl-Tartrat, Tridecylsalicylat, Lauryllactat, Diethyladipat, Capryl-/Capric-Triglyceride, Diisobutyladipat, Diisopropyladipat, Diethylhexyladipat, Diethylhexylsuccinat, Propandiol-Dicaprylat, Propylenglykol-Dicaprylat/Dicaprate, Isopropyl-Lauroylsarcosinat, Propylenglykol-Dibenzoat, Hydroxydimethoxybenzylmalonat, Phenoxyethylcaprylat, Isodecylsalicylat, Dimethylcapramid und Phenethylbenzoat

8. Zusammensetzung gemäß Anspruch 7, **dadurch gekennzeichnet, dass** sie mindestens die folgenden Lösungsvermittler entsprechend den INCI-Bezeichnungen umfasst: Dibutyladipat, Dicaprylylcarbonat und Diisopropylsebacat.

9. Zusammensetzung gemäß einem der vorstehenden Ansprüche, **dadurch gekennzeichnet, dass** die Lösungsvermittler zwischen 5 % und 80 % des Gesamtgewichtes der Zusammensetzung darstellen, bevorzugt zwischen 10 % und 70 %, vorzugsweise zwischen 15 % und 60 %.

10. Zusammensetzung gemäß einem der vorstehenden Ansprüche, **dadurch gekennzeichnet, dass** sie einen Sonnenschutzfaktor (SPF) von gleich oder höher als 30, bevorzugt von gleich oder höher als 50, vorzugsweise von gleich oder höher als 70 oder sogar von gleich oder höher als 100 aufweist.

11. Zusammensetzung wie gemäß einem der Ansprüche 1 bis 10 definiert zur Verwendung zum Schutz der Haut, der Lippen, der Haare und/oder der Schleimhäuten vor ultravioletter Sonnenstrahlung.

## Claims

1. A solar composition comprising:
- an aqueous phase;
- a fatty phase;
- hydroxypropyl starch phosphate (INCI);
- sodium starch octenylsuccinate (INCI);
- at least one triazine-derived solar filter;
the fatty phase represents between 20% and 70% by total weight of the composition,
the composition is free of emulsifier,
**characterized in that** the at least one triazine-derived solar filter is bis-ethylhexyloxyphenol methoxyphenyl triazine
or
**characterized in that** the weight ratio between hydroxypropyl starch phosphate (INCI) and sodium starch octenylsuccinate (INCI) is between 1/15 and 1/1, and **in that** the hydroxypropyl starch phosphate (INCI) and the sodium starch octenylsuccinate (INCI) represent between 0.5% and 15% by total weight of the composition.

2. Composition according to one of the preceding claims, **characterized in that** it comprises at least two, advantageously at least three, different triazine-derived solar filters.

3. Composition according to one of the preceding claims, **characterized in that** it comprises at least the triazine-derived solar filters corresponding to the following INCI names: bis-ethylhexyloxyphenol methoxyphenyl triazine and diethylhexyl butamido triazone, advantageously bis-ethylhexyloxyphenol methoxyphenyl triazine and diethylhexyl butamido triazone and ethylhexyl triazone.

4. Composition according to one of the preceding claims, **characterized in that** the at least one triazine-derived solar filter represents between 2% and 50% by total weight of the composition.

5. Composition according to Claim 4, **characterized in that** the at least one triazine-derived solar filter represents more than 10% by total weight of the composition, advantageously more than 12%, preferably more than 15%.

6. Composition according to one of the preceding claims, **characterized in that** it is in the form of a cream gel, advantageously a stable cream gel.

7. Composition according to one of the preceding claims, **characterized in that** it also comprises at least two solubilizers chosen from the group comprising the compounds corresponding to the following INCI names: dibutyl adipate, dicaprylyl carbonate, diisopropyl sebacate, caprylyl caprylate/caprate, dicaprylyl ether, cocoyl caprylate, C12-15 alkyl benzoate, propylheptyl caprylate, butylene glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, caprylic/capric triglycerides, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide and phenethyl benzoate.

8. Composition according to Claim 7, **characterized in that** it comprises at least the following solubilizers corresponding to the INCI names: dibutyl adipate, dicaprylyl carbonate and diisopropyl sebacate.

9. Composition according to one of the preceding claims, **characterized in that** the solubilizers represent between 5% and 80% by total weight of the composition, advantageously between 10% and 70%, preferably between 15% and 60%.

10. Composition according to one of the preceding claims, **characterized in that** it has a sun protection factor (SPF) greater than or equal to 30, advantageously greater than or equal to 50, preferably greater than or equal to 70, or even greater than or equal to 100.

11. Composition as defined in one of Claims 1 to 10, for use in protecting the skin, the lips, the hair and/or the mucous membranes against ultraviolet solar radiation.
